(19) Europäisches Patentamt
European Patent Office
Office européen des brevets

(11) EP 2 468 701 A1

(12) EUROPEAN PATENT APPLICATION

(43) Date of publication:
27.06.2012 Bulletin 2012/26

(21) Application number: 10196661.2

(22) Date of filing: 22.12.2010

(51) Int Cl.:
*C07C 1/32* (2006.01) *C07C 15/14* (2006.01)
*B01J 21/18* (2006.01) *B01J 23/44* (2006.01)
*C07B 37/04* (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: **Université Catholique de Louvain**
**1348 Louvain-la-Neuve (BE)**

(72) Inventors:
- **Gaigneaux, Eric**
**1450 Chastre (BE)**
- **Jacquemin, Marc**
**5580 Rochefort (BE)**
- **Hauwaert, Damien**
**1400 Nivelles (BE)**

(74) Representative: **Cabinet Plasseraud**
**52, rue de la Victoire**
**75440 Paris Cedex 09 (FR)**

(54) **Method of carrying out Suzuki-Miyaura CC-coupling reactions**

(57) The present invention is directed to a method of carrying out Suzuki-Miyaura CC-coupling reactions, comprising reacting an aryl halide with an aryl boronic acid in an organic solvent in the presence of a carbon supported palladium catalyst and a base, wherein said reactions are carried out at constant pH. The invention is also directed to a palladium on carbon caralyst suitable for catalyzing Suzuki-Miyaura CC-coupling reactions.

EP 2 468 701 A1

## Description

The present invention is directed to a method of carrying out C-C coupling reactions in the presence of a carbon supported palladium (Pd/C) catalyst and a base.

## BACKGROUND OF THE INVENTION

Chemical reactions aiming to couple carbon atoms are important methodologies for the preparation of organic molecules. These reactions have recently emerged and are often a crucial step in the synthesis of many molecules, especially for the synthesis of pharmaceuticals such as Vancomycine (antibiotic), Steganone, Steganacine (anticancer) or Korupensamine (antimalarial). These reactions are also essential for the synthesis of pesticides (Boscalid) and materials such as liquid crystals, organic conductive materials and semi-conductors. The demand for these molecules is getting more and more important.

Suzuki-Miyaura's reaction was published at first in 1979 by the 2010 nobel prize winner Akira Suzuki et al. (Miyaura N., Yamada K., Suzuki A., Tetrahedron Lett. 20, 1979, 3437). It creates an aryl-aryl bond in the presence of a palladium catalyst. It allows the combination of acids or vinyl esters or arylboronics via the boronate group with vinyl or aryl halides (Felpin F.-X., Ayad T. and Mitra S., Eur. J. Org. Chem., 2006, 2679). The reactivity of the aryl halide depends very strongly on the nature of the halides: I> Br» Cl. The iodides and bromides are usually used, on the other hand the chlorides are less reactive but more and more research is carried out about these molecules (Felpin F.-X., Ayad T. and Mitra S., Eur. J. Org. Chem., 2006, 2679; Simeone J.P., Sowa Jr. J.R., Tetrahedron 63, 2007, 12646-12654).

Suzuki-Miyaura's reaction is carried out in an organic solvent in the presence of a base, which is added at once the beginning of the reaction and consumed as the reaction goes on.

Suzuki-Miyaura's reaction is one of the most popular reactions for the production of biaryls for several reasons: (i) using mild conditions; (ii) using of boron compounds which are stable, available and have a low toxicity and (iii) a wide range of substrates with various functional groups can be used (Felpin F.-X., Ayad T. and Mitra S., Eur. J. Org. Chem., 2006, 2679; Kotha S., Lahiri K., Kashinath D., Tetrahedron, 58, 2002, 9633).

Suzuki-Miyaura's reaction is traditionally carried out with homogeneous palladium catalysts. These catalysts are soluble complexes of palladium associated to ligands of the arylphosphine type such as the tetrakis(triphenylphosphine)palladium ($Pd(PPh_3)_4$) (Lu G., Franze R., Zhanga Q., Xua Y., Tetrahedron Lett. 46, 2005, 4255; Amatore, C., Pflüger, F. Organometallics 9, 1990, 2276). Good yields are obtained with these ligands but they often need a big quantity of catalysts (1 to 10 mol %).

The formation of carbon-carbon bonds under homogeneous catalysis has a high potential. Unfortunately, the use of soluble complexes of palladium shows important drawbacks. Firstly, it is very difficult to recover the homogeneous catalysts which are very expensive and it is also difficult to separate them from the reactants and products. Therefore, this type of catalyst cannot be reused. Product contaminations by traces of dissolved catalyst remaining after separation can also arise. This point is particularly important in the synthesis of pharmaceutical molecules for which the residual metal tolerance is very low (less than 5 ppm). Gradually, organic chemistry is turning towards reusable heterogeneous catalysts for the economic and efficient use of raw materials.

Heterogeneous catalysts have been developed to avoid the problems mentioned previously. The main advantages of those catalysts are that they can be recovered by simple filtration at the end of the reaction and that there is no more product contamination by metals. The catalyst can also be recycled for further reactions. The most common heterogeneous catalyst used for the Suzuki-Miyaura reaction is palladium supported on activated carbons (Pd/C) (Felpin F.-X., Ayad T. and Mitra S., Eur. J. Org. Chem., 2006, 2679; Simeone J.P., Sowa Jr. J.R., Tetrahedron 63, 2007, 12646-12654; Lu G., Franze R., Zhanga Q., Xua Y., Tetrahedron Lett. 46, 2005, 4255). However, the activity of those catalysts is very low.

As a summary, there is still a stringent need for improvements of the Suzuki-Miyaura reaction that combine the advantages of homogenous palladium catalysts and of supported palladium catalysts.

## SUMMARY OF THE INVENTION

After long and intensive research, the present inventors found that the activity of palladium on carbon (Pd/C) catalysts in Suzuki-Miyaura reactions is significantly improved when the reactions are carried out at constant pH.

In a general aspect, the invention thus provides a method of carrying out Suzuki-Miyaura reactions, comprising reacting an aryl halide with an aryl boronic acid in an organic solvent in the presence of a carbon supported palladium catalyst and a base, characterized in that said reactions are carried out at constant pH.

In another aspect, the present invention provides a palladium on carbon catalyst, wherein the palladium is supported on a low specific surface carbon having a specific surface of less than 500 $m^2/g$, preferably less than 200 $m^2/g$, more preferably about 100 $m^2/g$.

## DETAILED DESCRIPTION OF THE INVENTION

As noted above, the invention relates to a method of carrying out Suzuki-Miyaura reactions, comprising reacting an aryl halide with an aryl boronic acid in an organic solvent in the presence of a carbon supported palladium catalyst and a base, characterized in that reaction is carried out at constant pH.

Advantageously, the constant pH is from 8 to 12, preferably from 10 to 11, more preferably about 10.6.

Traditionally, Suzuki-Miyaura reactions are carried out at decreasing pH as the base is added at once at the beginning of the reaction and then consumed as the reaction goes on. Working at constant pH under heterogenous catalysis improves the conversion rate and the selectivity of the Suzuki-Miyaura reaction compared to working at decreasing pH described in literature The selectivity is defined as the ratio between the number of moles of 4-methylbiphenyl produced and the number of moles of 4-bromotoluene converted. The invention thus provides a method of carrying out Suzuki-Miyaura reactions that combines the advantages of heterogeous catalysis with those of homogenous catalysis: easy recovery (e.g. by filtration) and recycling of the catalyst due to the use of a supported catalyst and increased activity of the catalysts leading to higher selectivity and conversion rates compared to traditional heterogenous catalysis.

In order to maintain a constant pH, the base is not, as usual, added at once at the beginning of the reaction, but continuously. The base is preferably an inorganic base. Suitable inorganic bases include, but are not limited to $K_2CO_3$, $Na_2CO_3$, $Cs_2CO_3$, NaOH, KOH, $NaHCO_3$, $Na_3PO_4$, and KF. In a preferred embodiment, the base is selected from the group consisting of $K_2CO_3$, NaOH, KOH, $Na_2CO_3$, and $NaHCO_3$.

A wide variety of organic solvents can be used for Suzuki-Miyaura reactions. Suitable organic solvents include, but are not limited to, DMF (dimethylformamide), DME (dimethoxyethane), DMA (dimethylacetamide), NMP (N-methylpyrrolidone), THF (tetrahydrofuran), toluene, methanol, ethanol, isorpropanol, n-butanol, water, and mixtures thereof.

The carbon support is either a high specific surface carbon or a low specific surface carbon. Within the meaning of the present invention, high specific surface carbons are those having a specific surface of more than 500 $m^2/g$, preferably more than 1000, and low specific surface carbons are those having a specific surface of less than 500 $m^2/g$, preferably less than 200 $m^2/g$, more preferably about 100 $m^2/g$.

In one embodiment, the carbon support is a high specific surface carbon as defined above, preferably activated carbon.

In another embodiment, the carbon support is a low specific surface carbon as defined above, preferably carbon black. Carbon black [C.A.S. NO. 1333-86-4] is virtually pure elemental carbon in the form of colloidal particles that are produced by incomplete combustion or thermal decomposition of gaseous or liquid hydrocarbons under controlled conditions. Carbon black is a form of amorphous carbon that has a low specific surface compared to that of activated carbon.

The catalysts used in the method of the invention can be prepared according to the methods known in the art, such as wet impregnation.

As already mentioned above, the invention also pertains to a carbon supported palladium catalyst, wherein the palladium is supported on a low specific surface carbon having a specific surface of less than 500 $m^2/g$, preferably less than 200 $m^2/g$, more preferably 80 to 100 $m^2/g$. In a preferred embodiment, the low specific surface carbon is carbon black. The main advantage of carbon black is its low specific surface and thus its larger pores than activated carbon. The larger pores reduce or even avoid problems of diffusion.

The present invention will be better understood with reference to the following examples. These examples are intended to representative of specific embodiments of the invention, and are not intended as limiting the scope of the invention.

## EXAMPLES

EXAMPLE 1 - Preparation of the catalysts

Two types of carbon were used as catalyst support:

- carbon LSS "Low Specific Surface" (carbon black: Printex U, Degussa, 970618214) having a specific surface area about 91 $m^2/g$ and
- carbon HSS "High Specific Surface" (activated carbon: Merck, 102518) having a specific surface area about 1263 $m^2/g$.

Before the impregnation, the supports were dried in air for 15h at 110°C.

0.3334g of palladium chloride ($PdCl_2$, Aldrich, 205885) was dissolved in an aqueous solution of HCl 0.1M. 4g of dried support was mixed with the impregnation solution for 10 min under magnetic stirring. Then, water was removed under reduced pressure in a rotavapor at 40°C. The recovered solid was dried at 110°C overnight. The catalysts were

synthesized with different loadings of Pd in mass comparing to the support: 1, 3, 5 and 10% for the LSS carbon and a loading of 5% for the HSS carbon. Catalysts are denoted Pd(x%)/C-y, where "x" represents the Pd weight loading in % and "y" is the support, LSS or HSS.

EXAMPLE 2 - Characterization of the Catalysts

The chemical composition of catalysts was measured by Inductively Coupled Plasma Atomic Emission Spectroscopy (ICP-AES) on an Iris Advantage apparatus from Jarrel Ash Corporation. The catalyst was dried at 110°C prior to measurement.

Textural analysis of the catalyst was carried out on a Micromeritics Tristar 3000 equipment using $N_2$ adsorption/desorption at liquid $N_2$ temperature, working with relative $P/P_0$ pressures in the range of $10^{-2}$ to 1.0. Before the measurements, 150mg of the samples were degassed at 150°C overnight under a vacuum (50 mTorr). The specific surface area was calculated from the amount of $N_2$ adsorbed by using 5 points with relative $P/P_0$ pressures between $5*10^{-2}$ and 0.3 (BET theory). BJH equations were used to determine the distribution of pores diameter and the total pore volume was assessed from the amount of nitrogen adsorbed at $P/P_0 = 0.98$.

X-ray diffraction (XRD) analysis were performed on the fresh catalyst on a Siemens D5000 diffractometer using the $K_\alpha$ radiation of Cu ($\lambda$=1.5418 Å). The 2$\theta$ range was scanned between 5 and 90° at a rate of 0.02°.$s^{-1}$. Identification of the crystalline phases was carried out using the ICDD-JCPDS database.

Surface characterization of the fresh catalyst was done by X-ray photoelectron spectroscopy (XPS) measurements on a Kratos Axis Ultra spectrometer (Kratos Analytical - Manchester - UK) equipped with a monochromatized aluminium X-ray source (powered at 10mA and 15KV).

The pressure in the analysis chamber was around $10^{-6}$ Pa. The analyzed area was 700$\mu$m x 300$\mu$m. The pass energy of the hemispherical analyser was set at 40eV. Charge stabilisation was achieved by using the Kratos Axis device. The electron source was operated at a filament current of 1.8 A and a bias of -1.1 eV. The charge balance plate was set at 2.8 eV. The sample powders were pressed into small stainless steel troughs mounted on a multi specimen holder. The following sequence of spectra was recorded: survey spectrum, C 1s, O 1s, A1 2p and Pd 3d and C 1s again to check the stability of charge compensation in function of time and the absence of degradation of the sample during the analyses. The binding energy (BE) values were referred to the C-(C,H) component of the C 1s peak fixed at 284.8eV. The spectra were decomposed with the CasaXPS program (Casa Software Ltd., UK) with a Gaussian/Lorentzian (70/30) product function. Molar fraction were calculated using peak areas normalised on the basis of acquisition parameters, sensitivity factors and transmission factors provided by the manufacturer.

Dispersion of Pd was determined by using carbon monoxide chemisorption. The CO chemisorption measurements were conducted at 35°C using a *Micromeritics Pulses Chemisorb 2700* apparatus equipped with a TCD detector. The sample (150 mg) was reduced at 400°C under a pure flow of Hydrogen (Praxair, 99.999%) for 2 hours and then flushed for 1 hour under He and finally cooled down to 35°C. Several injections of a known volume of CO (185$\mu$l) are sent on the samples. The apparatus gives a peak area which corresponds to the non-adsorbed CO. When there is no more adsorption of CO, the value of the peak area corresponds to the volume of the injection loop. By simple subtraction, the volume of CO which was adsorbed at each injection on palladium was calculated. Thereafter, those values were added for all injections of CO to obtain the total volume of CO adsorbed. This total is corrected so as to adjust the standard conditions of pressure and temperature. The stoechiometry of adsorption is equaled to one adsorbed CO molecule per atom of active metal (Pd).

The quantity of CO chemisorbed is related to the dispersion (D) with the following equation:

$$D = \frac{\sum \Delta S.V_{CO}.MM.A}{S_{\max}.B.m}$$

With:

- $\Sigma \Delta S$ : the sum of the difference between the values of area measured before the saturation of the sample and the value of the maximal area which corresponds to the saturation,
- $V_{CO}$: the volume injected CO (1),
- A: a conversion factor (273/(298 x 22.4)) (mol.$l^{-1}$),
- MM: molar mass of the metal (106.42 g.$mol^{-1}$),
- $S_{max}$: area of the peak which corresponds to the volume of CO injected in one injection,
- B: loading of the metal deposed on the support (%),

- m: mass of the analyzed sample (g).

The particle size of the Pd on the catalysts is estimated by the following equation:

$$d(m) = \frac{500 \times MM}{\rho \times \sigma \times D}$$

- d: Particle size (m)
- *MM* : Molar mass of the metal (Pd: 106.42 $g.mol^{-1}$);
- ρ : Density of the metal (Pd: 12 x $10^6$ $g.m^{-3}$);
- σ : Surface of one mole of the metal (Pd: 47800 $m^2_{metal}.mol^{-1}$);
- D: Dispersion (%).

The results of chemical analyses performed on catalysts supported on carbons are presented in Table 1. Dispersions (%) and the Pd particles size (nm) are also presented.

**Table 1: Chemical composition of catalysts supported on carbons**

| Catalysts | Pd (wt. %) experimental | Dispersion (%) | Particles size (nm) |
|---|---|---|---|
| Pd(1%)/C-LSS | 0,8 | 6 | 15 |
| Pd(3%)/C-LSS | 2,7 | 7 | 13 |
| Pd(5%)/C-LSS | 5.4 | 7 | 13 |
| Pd(10%)/C-LSS | 8,3 | 7 | 13 |
| Pd(5%)/C-HSS | 4,4 | 6 | 15 |

Table 2 shows that the specific surface area (SSA) remains identical for the LSS support and for the catalysts prepared with this one, except for the Pd(10%)/C-LSS which shows a decrease. Unlike, the SSA decreases significantly for Pd(5%)/C-HSS as compared to the fresh support. The SSA developed by the micropores represents the major part of the total specific surface area.

**Table 2: Textural analysis (BET) of the carbon support and catalysts supported on activated carbon**

| Catalysts | Specific surface area ($m^2$/g) | Porous volume ($cm^3$/g) | Pores size (Å) | Microporous specific surface area ($m^2$/g) |
|---|---|---|---|---|
| C-LSS | 91 | 0,80 | 376 | 19 |
| Pd(1%)/C-LSS | 87 | 0,77 | 353 | 18 |
| Pd(3%)/C-LSS | 99 | 0,71 | 257 | 12 |
| Pd(5%)/C-LSS | 99 | 0,84 | 257 | 18 |
| Pd(10%)/C-LSS | 68 | 0,51 | 332 | 9 |
| C-HSS | 1263 | 0,51 | 22 | 718 |
| Pd(5%)/C-HSS | 1088 | 0,48 | 23 | 634 |

In XRD analysis, all samples were characterized by a broad band around 20-25° typical of amorphous solids. No trace of crystalline palladium was found. This fact does not suggest that the Pd is not in crystalline form but it is possible that crystalline domains are present but undetected because they are too small.

The XPS analysis (Table 3) can also estimate the accessibility of Pd on the catalysts by the exploitation of atomic ratios Pd/C and its oxidation state by the exploitation of the Pd 3d peak position. In each analysis, the palladium is in the oxidized form ($Pd^{2+}$). The presence of Cl in some samples can be explained by the fact that the Pd precursor used is the catalyst $PdCl_2$ and carbon-based catalysts were simply dried and not calcined, so chlorine could not be volatilized. The amount of Cl is related to the ratio Pd/C, which is quite logical because when we add more Pd, we consequently add more chlorine.

**Table 3 - Analysis of the carbon-based catalysts surface by XPS**

| Catalysts | O/C | Pd/C | Cl/C |
|---|---|---|---|
| C-LSS | 0,08 | 0,00 | 0,00 |
| Pd(1%)/C-LSS | 0,10 | 0,04 | 0,01 |
| Pd(3%)/C-LSS | 0,12 | 0,10 | 0,01 |
| Pd(5%)/C-LSS | 0,17 | 0,22 | 0,03 |
| Pd(10%)/C-LSS | 0,26 | 0,43 | 0,06 |
| C-HSS | 0,05 | 0,00 | 0,00 |
| Pd(5%)/C-HSS | 0,16 | 0,14 | 0,02 |

EXAMPLE 3 - Catalytic Tests

Suzuki-Miyaura's catalytic tests reactions

The five Pd/C catalysts prepared in Example 1 were tested in the following Suzuki-Miyaura test reaction. The reactants were 4-bromotoluene and phenylboronic acid. The desired product is 4-methylbiphenyl.

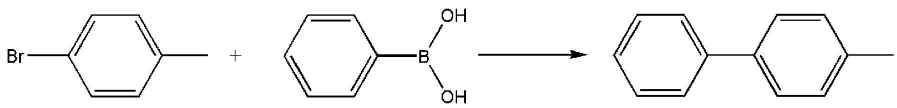

4-bromotoluene Phenylboronic acid 4-methylbiphenyl

**Coupling reaction**

A 5 neck round flask was placed in an oil bath. To reduce the loss of reagents by evaporation, a condenser is connected to the reactor. The reaction temperature was measured using a thermometer in contact with the reaction medium.

All catalysts were sieved and selected in the 100-200 μm granulometric fractions. The catalytic tests were carried out in the 5 neck flask with mechanical agitation (210 rpm). The solid reagents, 1.5000 g of 4-bromotoluene and 1.3256 g of phenylboronic acid, 0.1750 g of catalyst and 1.0810 g of biphenyl, the internal standard, were introduced first, followed by 60 ml of dimethylformamide (DMF). The flask was placed in a thermostatic oil bath and the reaction was performed under a nitrogen atmosphere. All catalytic tests have been realized by using a basic solution of $K_2CO_3$ (5g of $K_2CO_3$/100 ml of water tamponned with HCl at 10.6) and a reaction temperature of 95°C.

Once the working temperature (95°C) was reached, the desired amount (15m1) of $K_2CO_3$ solution was added manually using a syringe or an automatic titrator. The pH was monitored and the base added during the catalytic test so as to maintain the pH at 10.6.

The reaction begins when the $K_2CO_3$ solution was added. Samples (0.5 ml) were taken every 1min30 after adding the base until 10min30 and after a last aliquot was taken at 15min. Before analysis, samples are filtered (PTFE syringe filters, filtration threshold 0.45 μm) to remove the catalyst.

For quantification, an internal standard (biphenyl) was used and an appropriate calibration has been performed. Biphenyl was chosen as internal standard because it has a chemical structure very close to the desired reaction product, namely 4-methylbiphenyl. Tests were conducted to check that the internal standard did not react with any compounds present in the reaction, namely 4-bromotoluene, phenylboronic acid, 4-methylbiphenyl, the base and DMF. For the analysis of samples, the sample was diluted (1:9 vol) in dichloromethane (JANSSEN CHIMICA, 1134696) is conducted and analyzed by a CP-3800 gas chromatography (GC) apparatus from Varian equipped with an autosampler (CP-8200 Varian) on a column CP-Sil 5CB (50 m x 0.32mm x 0.4μm) Varian. The initial pressure is 15 psi in the column. The temperature program applied for the separation of reactants and products in the chromatography column is as follows: initial temperature 80°C is maintained 3min, and then a ramp of 30°C/min was applied up to 180°C. This temperature is maintained for 3 minutes. A ramp of 15°C/min is applied to 250°C for 3min. Finally, the temperature increases to 300°C with a ramp of 30°C/min. This temperature is maintained during 3min. The total duration of the program is 15.76 minutes. The conversion is defined as the ratio "moles of 4-bromotoluene converted/mole of 4-bromotoluene initial * 100 %". The selectivity for the 4-methylbiphenyl is defined as the ratio "moles of 4-methylbiphenyl produced/mole of 4-bromotoluene consumed * 100 %".

Test Results

The catalytic performances of the catalysts supported on the LSS carbon with different loadings (1, 3, 5, 10 %) have been measured. Figure 1 and Figure 2 show respectively the conversion of the 4-bromotoluene and the selectivity to 4-methylbiphenyl depending on the time for those catalysts. The results obtained are also much better than those found in the literature (Felpin F.-X., Ayad T. and Mitra S., Eur. J. Org. Chem., 2006, 2679; Simeone J.P., Sowa Jr. J.R., Tetrahedron 63, 2007, 12646-12654).

Regarding the comparison of a Pd/C-LSS and a Pd/C-HSS catalyst, the conversion of 4-bromotoluene reaches 100 % after the same time (6 min), but the activity of the Pd(5%)/C-LSS was higher at the beginning. On the contrary, the production of 4-methylbiphenyl was slightly higher for the Pd(5%)/C-HSS after 6 min of reaction. However after 1.5 min of reaction, the selectivity was the same.

It has been experimentally determined that using a constant pH of 10.6 was the optimum way to introduce the base during the reaction with the carbon-supported catalysts (Figures 6 and 7). This is accomplished through the use of an automatic titrator which maintains a constant pH by adding base to replace the one consumed during the reaction.

COMPARATIVE EXAMPLE

The Suzuki-Miyaura test reaction described in Example 3 was carried out using the Pd(5%)/C-LSS catalyst of example 1. In contrast to Example 3, 15 ml of $K_2CO_3$ solution (5g/100ml of water buffered with HCl at 10.6) were added at once at the beginning of the reaction.

The starting pH was close to 14 and decreased during the reaction. The conversions of both tests (constant pH versus a "classical test" in which the base was added at the beginning of the test and the pH decreased during the test) were identical after 6 min of reaction (Figure 3). However, the selectivity to 4-methylbiphenyl reached 100% after 6 min when the pH was kept constant at 10.6 (Figure 4), whereas the selectivity in the "classical test" below 90% after 6 min.

Hence, working at constant pH improves the selectivity of the test reaction while maintaining equivalent conversion rates.

## Claims

1. Method of carrying out Suzuki-Miyaura CC-coupling reactions, comprising reacting an aryl halide with an aryl boronic acid in an organic solvent in the presence of a carbon supported palladium catalyst and a base, **characterized in that** said reactions are carried out at constant pH.

2. The method according to claim 1, **characterized in that** the constant pH is from 8 to 12, preferably from 10 to 11, more preferably about 10.6.

3. The method according to claim 1 or 2, **characterized in that** the base is an inorganic base.

4. The method according to claim 3, **characterized in that** the base is selected from the group consisting of $K_2CO_3$, NaOH , KOH, $Na_2CO_3$, and $NaHCO_3$.

5. The method according to any one of the preceding claims, **characterized in that** the carbon support of the catalyst is selected from high specific surface carbons having a specific surface of more than 1000 $m^2$/g or low specific surface carbons having a specific surface of less than 500 $m^2$/g, preferably less than 200 $m^2$/g, more preferably about 100 $m^2$/g..

6. The method according to claim 5, **characterized in that** the carbon support is selected from high specific surface carbons, preferably activated carbon.

7. The method according to claim 6, **characterized in that** the carbon support is selected from low specific surface carbons, preferably carbon black.

8. A carbon supported palladium catalyst, wherein the palladium is supported on a low specific surface carbon having a specific surface of less than 500 $m^2$/g, preferably less than 200 $m^2$/g, more preferably 80 to 100 $m^2$/g.

9. The catalyst according to claim 8, **characterized in that** the carbon support is carbon black.

**10.** Use of the catalyst according to claim 8 or 9 in Suzuki-Miyaura CC-coupling reactions.

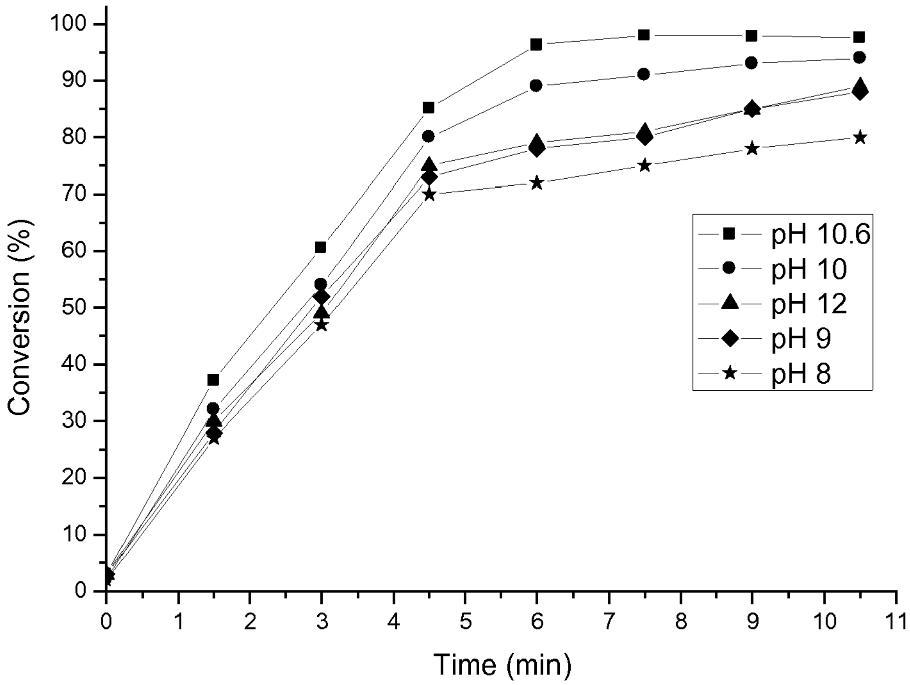
100
90
80
70
60
50
40
30
20
10
0
Conversion (%)
0
1
2
3
4
5
6
7
8
9
10
11
Time (min)
pH 10.6
pH 10
pH 12
pH 9
pH 8

**Fig. 1**

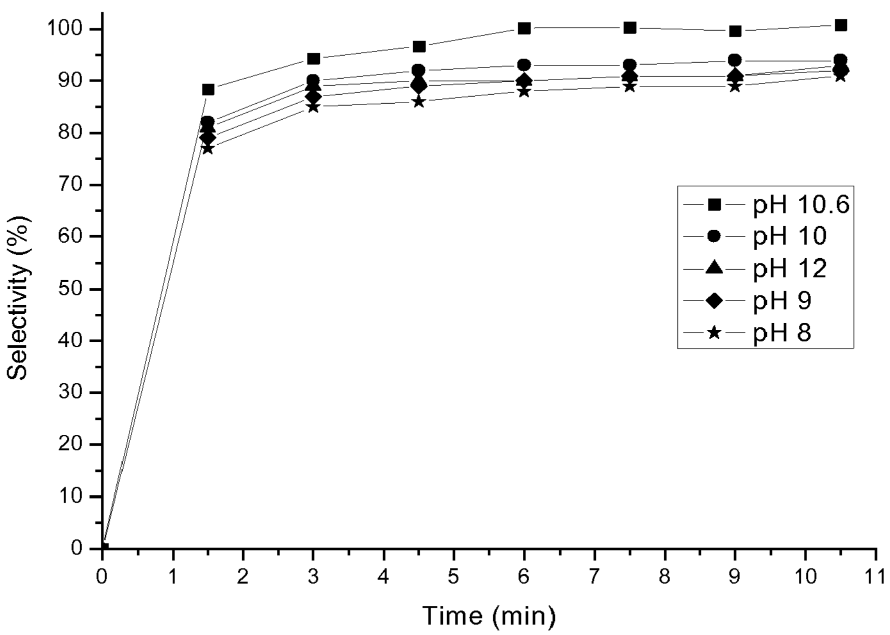
100
90
80
70
60
50
40
30
20
10
0
Selectivity (%)
0
1
2
3
4
5
6
7
8
9
10
11
Time (min)
pH 10.6
pH 10
pH 12
pH 9
pH 8

**Fig. 2**

Europäisches Patentamt
European Patent Office
Office européen des brevets

# EUROPEAN SEARCH REPORT

Application Number
EP 10 19 6661

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUILLEN E ET AL: "Pd-activated carbon catalysts for hydrogenation and Suzuki reactions", APPLIED CATALYSIS A: GENERAL, ELSEVIER SCIENCE, AMSTERDAM, NL, vol. 368, no. 1-2, 31 October 2009 (2009-10-31), pages 113-120, XP026641988, ISSN: 0926-860X, DOI: DOI:10.1016/J.APCATA.2009.08.016 [retrieved on 2009-08-19] * page 118, right-hand column - page 119 * | 1-7 | INV. C07C1/32 C07C15/14 B01J21/18 B01J23/44 C07B37/04 |
| A,D | F.-X. FELPIN: "Pd/C: An Old Catalyst for New Applications - Its Use for the Suzuki-Miyaura Reaction", EUR. J. ORG. CHEM., 8 March 2006 (2006-03-08), pages 2679-2690, XP002634398, * the whole document * | 1-7 | |
| A,D | LU G ET AL: "Palladium charcoal-catalyzed, ligandless Suzuki reaction by using tetraarylborates in water", TETRAHEDRON LETTERS, ELSEVIER, AMSTERDAM, NL, vol. 46, no. 24, 13 June 2005 (2005-06-13), pages 4255-4259, XP027281798, ISSN: 0040-4039 [retrieved on 2005-05-12] * the whole document * | 1-7 | TECHNICAL FIELDS SEARCHED (IPC): C07C B01J C07B |

-/--

~~The present search report has been drawn up for all claims~~

2

| Place of search | Date of completion of the search | Examiner |
|---|---|---|
| Munich | 28 April 2011 | Österle, Carmen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 19 6661

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A,D | SIMEONE ET AL: "Palladium on carbon as a precatalyst for the Suzuki-Miyuara cross-coupling of aryl chlorides", TETRAHEDRON, ELSEVIER SCIENCE PUBLISHERS, AMSTERDAM, NL, vol. 63, no. 51, 8 November 2007 (2007-11-08), pages 12646-12654, XP022336716, ISSN: 0040-4020, DOI: DOI:10.1016/J.TET.2007.10.007 * the whole document * ----- | 1-7 | |
| A | EP 1 186 583 A1 (DEGUSSA [DE]) 13 March 2002 (2002-03-13) * claims * ----- | 1-7 | |
| | | | TECHNICAL FIELDS SEARCHED (IPC) |

~~The present search report has been drawn up for all claims~~

2

| Place of search | Date of completion of the search | Examiner |
|---|---|---|
| Munich | 28 April 2011 | Österle, Carmen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................
& : member of the same patent family, corresponding document

EPO FORM 1503 03.82 (P04C01)

Europäisches Patentamt
European Patent Office
Office européen des brevets

Application Number
EP 10 19 6661

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  1-7

- [ ] The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION**
**SHEET B**

Application Number
EP 10 19 6661

The Search Division considers that the present European patent application does not comply with the requirements of unity of invention and relates to several inventions or groups of inventions, namely:

1. claims: 1-7

   Method of carrying out Suzuki-Miyaura CC-coupling reactions characterized in that said reactions are carried out at constant pH

   ---

2. claims: 8, 9

   A carbon supported palladium catalyst, wherein the palladium is supported on a low specific surface carbon

   ---

3. claim: 10

   Use of a palladium catalyst supported on a low specific surface carbon in the Suzuki-Miyaura CC-coupling

   ---

## ANNEX TO THE EUROPEAN SEARCH REPORT ON EUROPEAN PATENT APPLICATION NO. EP 10 19 6661

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2011

| Patent document cited in search report | Publication date | Patent family member(s) | Publication date |
|---|---|---|---|
| EP 1186583 A1 | 13-03-2002 | WO 0220433 A1 | 14-03-2002 |
| | | US 2003181748 A1 | 25-09-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MIYAURA N. ; YAMADA K. ; SUZUKI A.** *Tetrahedron Lett.,* 1979, vol. 20, 3437
- **FELPIN F.-X. ; AYAD T. ; MITRA S.** *Eur. J. Org. Chem.,* 2006, 2679
- **FELPIN F.-X. ; AYAD T. ; MITRA S.** *Eur. J. Org. Chem,* 2006, 2679
- **SIMEONE J.P. ; SOWA JR. J.R.** *Tetrahedron,* 2007, vol. 63, 12646-12654
- **KOTHA S. ; LAHIRI K. ; KASHINATH D.** *Tetrahedron,* 2002, vol. 58, 9633
- **LU G. ; FRANZE R. ; ZHANGA Q. ; XUA Y.** *Tetrahedron Lett.,* 2005, vol. 46, 4255
- **AMATORE, C. ; PFLÜGER, F.** *Organometallics,* 1990, vol. 9, 2276
- **SIMEONE J.P ; SOWA JR. J.R.** *Tetrahedron,* 2007, vol. 63, 12646-12654